# EUROPEAN PATENT SPECIFICATION

(11) **EP 3 084 439 B1**
(45) Date of publication and mention of the grant of the patent: **19.09.2018**
(21) Application number: 14824416.3
(22) Date of filing: 16.12.2014
(51) Int. Cl.: G01N 33/543, G01N 33/68

(54) **ANALYSIS OF ANTIBODIES**
ANALYSE VON ANTIKÖRPERN
ANALYSE D'ANTICORPS

(30) Priority: 20.12.2013 US 201361919156 P; 02.07.2014 US 201462019945 P
(43) Date of publication of application: 26.10.2016
(73) Proprietor: Phadia AB, 751 37 Uppsala (SE)
(72) Inventor: MOVÉRARE, Robert, S-751 37 Uppsala (SE); ERIKSSON, Camilla, S-751 37 Uppsala (SE); VENEMALM, Lennart, S-751 37 Uppsala (SE)
(74) Representative: Brann AB
(86) International application number: PCT/EP2014/077897
(87) International publication number: WO 2015/091438

(56) References cited:
- WO-A1-2010/003602
- WO-A1-2012/010290
- WO-A1-2013/132000
- US-A1- 2011 183 363
- STUBENRAUCH K ET AL: "Evaluation of a biosensor immunoassay for simultaneous characterization of isotype and binding region of human anti-tocilizumab antibodies with control by surrogate standards", ANALYTICAL BIOCHEMISTRY, ACADEMIC PRESS INC, NEW YORK, vol. 390, no. 2, 15 July 2009 (2009-07-15) , pages 189-196, XP026145499, ISSN: 0003-2697, DOI: 10.1016/J.AB.2009.04.021 [retrieved on 2009-04-18]
- LUNDKVIST M ET AL: "Characterization of anti-natalizumab antibodies in multiple sclerosis patients", MULTIPLE SCLEROSIS JOURNAL, vol. 19, no. 6, May 2013 (2013-05), pages 757-764, XP008175740,
- L X TIEFENAUER ET AL: "Antibody coating loo. using various avidin-biotin complexes employed to an enzyme immunoassay for estradiol", FRESENIUS Z ANAL CHEM, vol. 330, 1 January 1988 (1988-01-01), page 342, XP055180626,

## Description

### Technical Field

The present invention relates to the field of biochemical analysis, and more specifically to a method useful in the context of biopharmaceuticals.

### Background

Biopharmaceutics is the study of medical drugs produced using biotechnology. The first such substance approved for therapeutic use was biosynthetic insulin made via recombinant DNA technology. The main focus of attention after the insulin was to find other potential profit makers in the pharmaceutical industry, such as other recombinant proteins and monoclonal antibodies. Monoclonal antibody therapy includes the use of monoclonal antibodies that specifically bind to target cells in a patient, which then stimulate the immune system to attack those cells, for example in the treatment of cancer. It is possible to create a monoclonal antibody specific to almost any extracellular or cell surface target. Thus, there is a large amount of research and development currently being undertaken to create monoclonal antibodies for numerous serious diseases. A specific example is in the treatment of rheumatoid arthritis, where monoclonal antibodies suppress the response to tumor necrosis factor alpha (TNF-α), which is part of the inflammatory response. Another example is found in the treatment of multiple sclerosis, where monoclonal antibodies are raised against certain integrins on leukocytes whereby prevention of leukocyte infiltration through the blood-brain barrier is enabled.

However, not all patients respond as expected to biological drugs. Endogenous antibody formation against biological drugs, so called anti-drug antibodies (ADAs), is a major problem; as such ADAs may neutralize the action of the drug and even result in adverse reactions. Thus, the monitoring of ADAs has been considered important both from a medical standpoint and for health economic reasons. Consequently, the analysis of ADA formation has become an important part of new biological drug development and even a requirement from regulatory bodies.

Various assay formats have been described for the analysis of ADAs. They include cell-based assays for analysis of drug-neutralizing antibodies (NAbs) and chromatographic methods and surface plasmon resonance technique for analysis of drug-binding antibodies (BAbs). Most used however, are different variants of immunoassays for high-throughput screening and analysis of ADAs in a convenient and effective way.

In general terms, immunoassays described for the analysis of ADAs are bridging assays, where the multivalency of the ADA is utilized to complex biological drugs in solution and/or attached to a solid phase; antibody immunosorbent assays of sandwich type, for example enzyme-linked immunosorbent assays (ELISAs), which are based on biological drugs bound to a solid phase to which ADAs are allowed to bind and later detected; and reversed antibody assays, also known as indirect ELISA or fluid phase RIA, wherein all immunoglobulins of selected isotypes, irrespective of their antigen specificity, are allowed to bind to a solid phase by a catching reagent, such as an isotype-specific antibody or Protein A or Protein G, and in which the ADAs later are detected by their drug-binding capacity.

IgG4 antibodies have shown to be of specific importance to analyse separately in patients receiving biological drugs. Commercial products are available for clinical measurements of IgG4 antibodies, such as ImmunoCAP™ Specific IgG4 (available through www.phadia.com) which is used extensively in clinical studies of allergic disease.

Rheumatoid factor (RF) is the name of autoantibodies defined as antibodies against the Fc portion of human IgG. RF is often evaluated in patients suspected of having any form of arthritis and is part of the usual disease criteria of rheumatoid arthritis. Although the isotype of RF usually is IgM, any isotype of immunoglobulins, including IgG, has been described.

WO 2009/077127 (F. Hoffmann-La Roche AG) relates to a distinguishing assay, wherein an antibody against a drug antibody is determined in a sample using an immunoassay comprising a capture drug antibody, which is the drug antibody conjugated to a solid phase, and a tracer drug antibody, which is the drug antibody conjugated to a detectable label. The capture drug antibody is contacted separately with (i) the sample; (ii) the sample to which the drug antibody in monomeric form has been added; and (iii) the sample, to which the drug antibody in oligomeric form has been added. Determination of antibody against the drug antibody is made by a positive immunoassay in (i), and negative immunoassay in (ii) and (iii).

Assay background is well known to cause problems in any immunoassay development. In immunosorbent assays for analysis of antibodies, interference of total immunoglobulin may generate false positive results and low sensitivity, as the antigen-specific antibodies are usually present as a small fraction only of the total immunoglobulin in a sample. Due to the high concentration of IgG in serum and plasma, IgG interference is a frequently occurring problem in IgG antibody immunosorbent assays. Another interference problem is caused by interactions between the crystallizable fragments (Fc fragments) of antibodies, hence known as Fc-Fc interactions. For example, in an immunosorbent assay targeting ADAs of isotype IgG4, Fc-Fc interaction may occur between IgG4 and other IgG molecules used as antigen attached to a solid phase, for example therapeutic IgG antibodies.

One way to overcome the Fc-Fc interaction in antibody immunosorbent assays is to simply avoid having Fc fragments, and instead use purified enzyme-cleaved F(ab')2 fragments, or Fab fragments of the IgG molecules, as antigen attached to the solid phase. Alternatively, instead of using the antibody immunosorbent assays format, reversed antibody assays could be used where all IgG4 in the sample is bound by IgG4-specific reagents attached to a solid phase whereafter labelled whole IgG or fragments thereof is added as detection agent, or reagent.

WO 2012/02774 (Roche Diagnostics GmbH) relates to an assay for measurement of antibodies binding to a therapeutic monoclonal antibody, wherein Fab fragments of the therapeutic monoclonal antibodies bound to a surface are incubated with a sample comprising antibodies against the therapeutic monoclonal antibody.

Rispens et al (Rispens T, Ooievaar-De HP, Vermeulen E, Schuurman J, van der Neut KM, Aalberse RC. Human IgG4 binds to IgG4 and conformationally altered IgG1 via Fc-Fc interactions. J Immunol 2009; 182:4275-81) have investigated the physicochemical aspects of Fc-Fc interactions and discuss possible implications from a biological and medical viewpoint.

Lundkvist M et al: "Characterization of anti-natalizumab antibodies in multiple sclerosis patients", MULTIPLE SCLEROSIS JOURNAL, Vil. 19, no. 6, May 2013 (2013-05), pages 757-764, XP008175740 discloses a sandwich immunoassay for ADAs of IgG1, IgG2, IgG3 and IgG4 and IgM isotypes comprising coating a plate with the drug natalizumab, adding sample and contacting it with labelled isotype-specific antibodies.

Thus, there is a need in this field of novel assay formats, whereby unspecific binding caused e.g. be Fc-Fc interactions may be reduced.

### Summary of the Invention

The present invention relates to a method as defined in independent claim 1 and to uses as defined in independent claims 11 and 12.

Other embodiments, advantages and details will appear from the dependent claims and from the detailed description that follows below.

### Brief description of the Drawings

Figure 1 is a schematic figure showing the principle of a known format of an antibody binding immunosorbent assay for ADAs.
Figure 2 is a schematic figure illustrating the problem of increased backgrounds in an IgG4 antibody immunosorbent assay due to Fc-Fc interaction.
Figure 3 is a schematic figure showing how a linker can be used according to the invention to displace the IgG molecule from the proximity of the solid phase in an IgG4 antibody immunosorbent assay.
Figure 4 is a schematic figure showing how an antibody target can be used as linker according to the invention to displace the IgG molecule from the proximity of the solid phase in an IgG4 antibody immunosorbent assay.
Figure 5 shows four ADA negative samples, assay diluent and one ADA positive sample tested according to conventional techniques, as described in Experimental part below.
Figure 6 shows seven serum samples defined as ADA positive for infliximab using other assays (the first appearing seven bars, from the left), seven samples defined as ADA negative (the following seven bars), and assay diluent (the last bar) analyzed for IgG4 antibodies to infliximab using the ImmunoCAP™ Specific IgG4 assay.

### Definitions

The term "IgG molecule" means herein any molecule which comprises at least one Fc region, or at least one constant domain capable of causing unspecific binding, of an immunoglobulin.

The phrase "capable of binding" as used herein should be understood in its broadest sense i.e. as capable of participating in binding; or capable of being bound. For example, the phrase "a molecule capable of being bound by an antibody" includes herein that an antibody binds said molecule.

The term "biopharmaceutical" is used interchangeably with "biological drugs" which may be based on antibodies also known as therapeutic antibodies and includes but is not limited to whole mouse monoclonal antibodies, such as muromonab-CD3 and tositumomab, chimeric human/mouse monoclonal antibodies, such as infliximab and cetuximab, humanized monoclonal antibodies, such as natalizumab and omalizumab, fully human monoclonal antibodies, such as adalimumab and golimumab; target-binding regions of monoclonal antibodies; fusion proteins composed of parts of antibodies such as Fc regions; and other molecules such as cytokine receptors, e.g. etanercept.

The term "anti-drug antibody" or ADA was explained in broad term under "Background" above, and is used herein in that broad sense. Thus, an ADA may have been raised against a drug comprising one or more parts or domains of an antibody, such as one or more immunoglobulin Fc gamma chain(s). Alternatively, an "IgG drug" antibody against an ADA as used herein may be, or comprise, a fusion protein, wherein part(s) of one or more antibodies and/or antigens are present.

In the present application, the terms "raised" and "directed" are used interchangeably in relation to antibodies to describe which molecule or compound the antibody is directed and specific against.

### Detailed description of the Invention

In a first aspect, the present invention is a method as defined independent claim 1.

The endogenously formed antibodies are directed against IgG. Further, the endogenously formed antibodies are ADAs, which ADA may be against a recombinant protein, a fusion protein or a protein fragment, obtained by endogenous formation in a patient receiving treatment with a biological drug or *in vitro.* The ADA may also be one or more pre-existing antibodies in a patient not receiving treatment with a biological drug.

As the skilled person will appreciate, the class of the ADA will depend on the nature of the biological drug or biopharmaceutical against which it has been raised, as well as the drug formulation and administration. In a specific embodiment, the ADAs are against a common group of biopharmaceuticals, such as an IgG drug, e.g. IgG1.

As the skilled person will appreciate, the ADA may be an antibody of any isotype, such as immunoglobulin A, D, E, G or M. In the method of the invention, ADAs of IgG4 isotype are analyzed.

Alternatively, antibodies against other substances than biopharmaceuticals are analysed. Thus, in this embodiment, the endogenously formed antibodies are so called RF, which is relevant in rheumatoid arthritis.

As appears from the above, one aspect of the invention is the use of the claimed method in the context of biopharmaceuticals. Thus, the molecule capable of binding endogenously formed antibodies molecule may be a therapeutic IgG antibody. The IgG is advantageously human, but may be of other origin such as mouse or the like. In one embodiment, the IgG molecule is a monoclonal antibody, and in an alternative embodiment, the IgG molecule is a polyclonal antibody.

The molecules capable of binding endogenously formed antibodies are IgG molecule(s). In one embodiment, the molecules capable of binding said endogenously formed antibodies are selected from the group consisting of IgG1, IgG2, IgG3 and IgG4.

In one embodiment, the molecules capable of binding endogenously formed antibodies are complete antibodies such as complete IgG. In an alternative disclosed aspect, the molecules capable of binding endogenously formed antibodies are fragments of antibodies, such as one or more Fc region of an antibody, such as IgG. In a specific disclosed aspect, the molecules capable of binding endogenously formed antibodies are one or more constant domain(s), such as one or more constant domain(s) of IgG.

The present inventors have shown that the attachment of the molecules capable of binding said endogenously formed antibodies to a solid phase via a linker can reduce unspecific binding as is often caused by Fc-Fc interactions in an immunoassay. The distancing of said molecules capable of binding said endogenously formed antibodies from the proximity of the solid phase reduces the extent of such solid phase-induced changes in the structure of the IgG attached molecule that facilitate the above-discussed undesired Fc-Fc interactions.

The linker used in the present method may be any entity that achieves this purpose, such as an organic molecule, an amino acid, a peptide, a protein or molecule of protein origin, a monosaccharide, an oligosaccharide or a polysaccharide. It may be a synthetic linker, such as poly-L-lysine, a dendrimer, an oligomer of ethyleneglycol, a polymer of ethyleneglycol, or a protein such as human serum albumin. Alternatively, the linker may be any other molecule that enables the advantageous reduction and/or even elimination of interference provided by the present invention as described in more detail elsewhere in the present application. Thus, in one embodiment, the linker is an organic molecule, an amino acid, a peptide, a protein or a molecule of protein origin, a monosaccharide, an oligosaccharide or a polysaccharide.

In one embodiment, the linker may be provided as part of the solid phase, and IgG or other molecules are then conjugated to the solid phase. Thus, molecules pendant from the solid phase will act as linkers providing the advantageous effect according to the invention.

Thus, in one embodiment of the present method, the linker is formed by the covalent coupling of the molecule(s) capable of binding endogenously formed antibodies to molecules extending from a solid phase comprised of natural polymer, such as cellulose.

The skilled person may design an assay useful in the method according to the invention by specifically constructing or choosing a linker which provides certain desired advantages. Thus, in one embodiment, the molecule(s) capable of binding endogenously formed antibodies is at least one antibody with known specificity and the linker is its target ligand.

The linker used in the present method may be provided by using the well-known streptavidin/biotin coupling system, and more specifically by attaching the biotin to the IgG molecule following well known procedures, and attaching the streptavidin to the solid phase by adsorption, as is as commonly used in ELISA, or by covalent coupling, as is commonly used in ImmunoCAP™ tests. Thus, here the streptavidin molecule will act as the linker that binds the biotin-labelled IgG molecule and positions the IgG molecule out from the proximity of the solid phase, which has been shown according to the invention to provide unexpected advantages in terms of reduced interference, especially a reduced level of undesired Fc-Fc interactions. Accordingly, in a specific embodiment of the present method, the molecule(s) capable of binding endogenously formed antibodies have been labeled with biotin and the linker is streptavidin.

The technology for making fusion proteins including two or more elements from different proteins, or different parts of proteins, is well known and commonly employed in this field. According to the present invention, a fusion protein may be constructed to include elements which are useful in the present method.

Thus, in a one embodiment, the molecule(s) capable of binding endogenously formed antibodies is at least one fusion protein between a ligand-binding molecule and the Fc region of an IgG molecule, and the linker is its target ligand.

In one embodiment of the present method, the labeled IgG4-specific antibody capable of binding the endogenously formed antibodies is a monoclonal or polyclonal antibody.

The endogenous antibody analysed according to the present invention may have been raised in an individual receiving therapy using a biological drug, such as a therapeutic antibody. Thus, in one embodiment, the solution is a biological sample, such as blood. The present innovation describes a new principle or method, which is useful in the analyses of antibodies, namely IgG4 molecules. An antibody immunosorbent assay according to the invention can therefore be used for making clinical decisions for patient care as well as for use in new biological drug development.

Consequently, in one embodiment, the present invention relates to the use of a method according to the invention to monitor a patient's antibody response to a therapeutic antibody.

Alternatively, as indicated above, the endogenously formed antibody may be RF. Accordingly the method according to the invention can be used to evaluate patients suspected of having any form of arthritis, in diagnosis and/or disease prognosis.

The present invention may be used in an immune assay as described above in drug development.

The invention may be used to analyze the presence of RF in a patient sample.

The invention may be used in any of the following formats: an assay of sandwich type (the claimed invention uses a sandwich format), such as Radio ImmunoAssay (RIA), Enzyme ImmunoAssay (EIA), Enzyme-Linked ImmunoSorbent Assay (ELISA), Luminescence ImmunoAssay (LIA), or Electrochemi-luminescence (ECL) assay; a multiplex assay, such as a multiarray-based assay or a particle-based assay; a lateral flow-based assay, e.g. for point-of-care use; a proximity assay; an agglutination assay; a turbometric assay; or a nephelometric assay.

The ECL format may be suitable as it may omit washing step(s). The kit may be designed for analysis of ADAs or RF in a system that allows simultaneous detection/measurement of more than one analyte.

One aspect of the kit is a point-of-care (POC) test based on a capillary-driven microporous membrane, such as a nitrocellulose membrane, for the analysis of ADAs or RF using the method described above.

Another aspect of the kit is for proximity immunoassay where the drug is attached to one type of solid phase such as beads, denoted donor beads, and the detection reagent is attached to another type of solid phase, such as beads, denoted acceptor beads. Thus, such a kit comprises donor beads, acceptor beads, means for generating a signal when the beads are complexed by the analyte. An advantage of this format is that washing steps may be omitted.

The kit according to the disclosure may include written instructions for its use to detect ADAs or RF; a suitable number of appropriately sized containers; solvents; reagents; and the like.

### Detailed description of the drawings

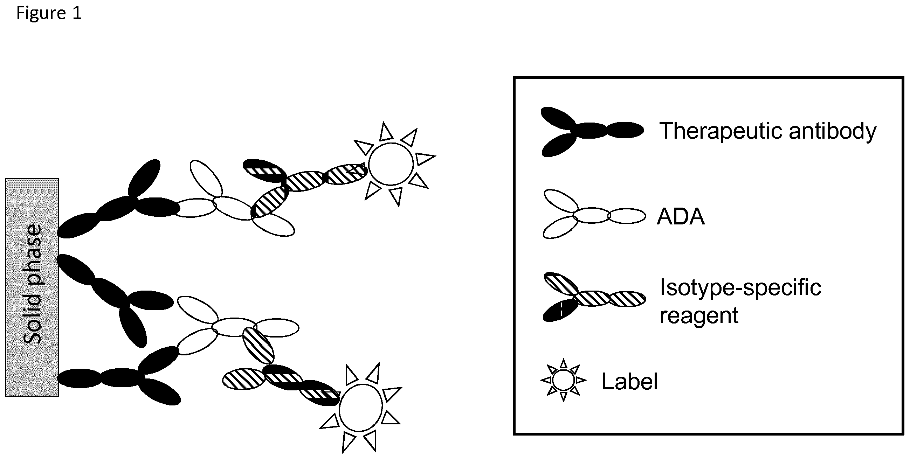

Figure 1 is a schematic figure showing the principle of an antibody binding immunosorbent assay, also known as solid-phase ELISA, for anti-drug antibodies (ADAs).

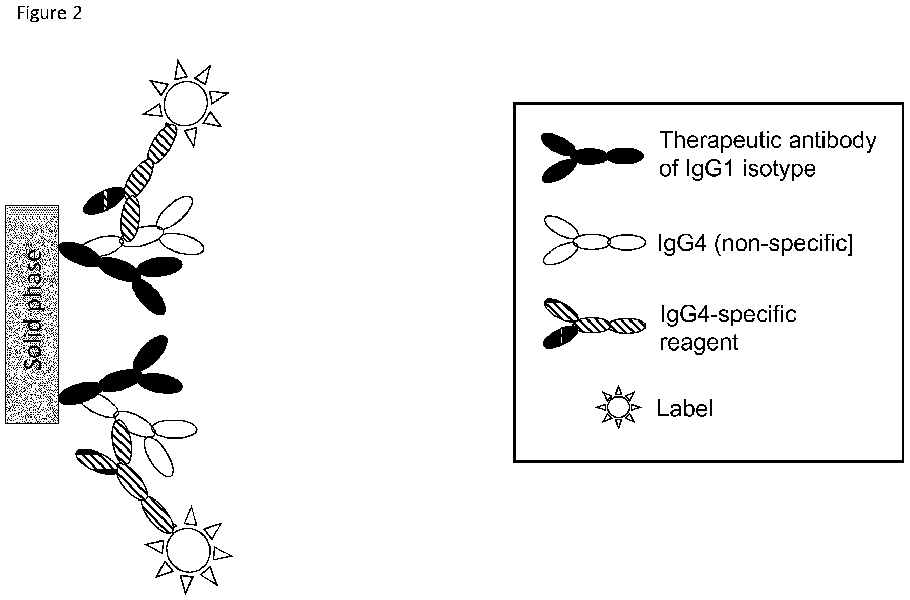

Figure 2 is a schematic figure illustrating the problem of increased backgrounds in an IgG4 antibody immunosorbent assay due to Fc Fc interaction between an IgG molecule, which may be a therapeutic antibody of IgG1 isotype, in close proximity to the solid phase and IgG4 in an ADA negative sample.

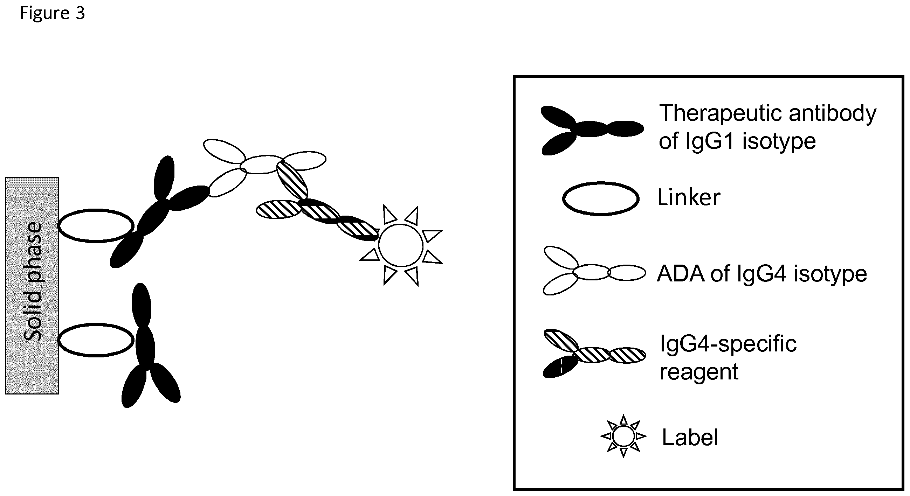

Figure 3 is a schematic figure showing how a linker can be used according to the invention to displace the IgG molecule, which may be a therapeutic antibody of IgG 1 isotype, from the proximity of the solid phase of an IgG4 antibody immunosorbent assay.

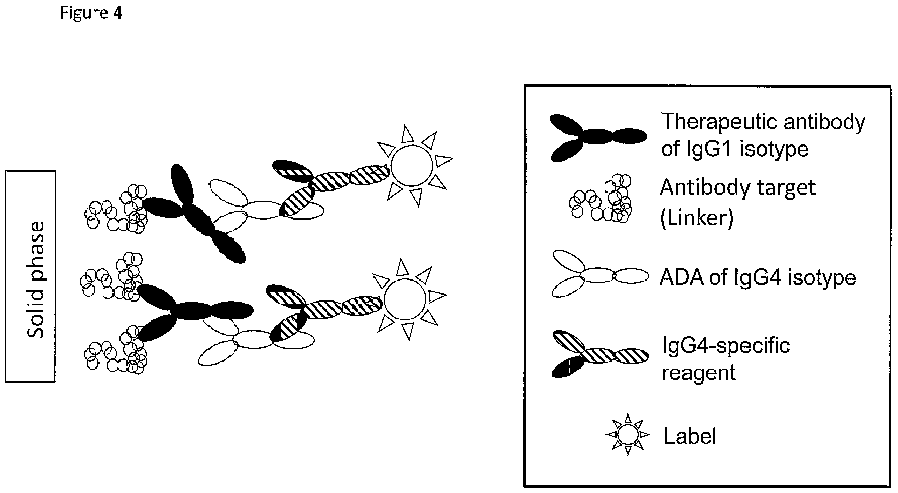

Figure 4 is a schematic figure showing how an antibody target, which advantageously is an antigen, can be used as linker to displace the IgG molecule, which may be a therapeutic antibody of IgG1 isotype, from the proximity of the solid phase in an IgG4 antibody immunosorbent assay.

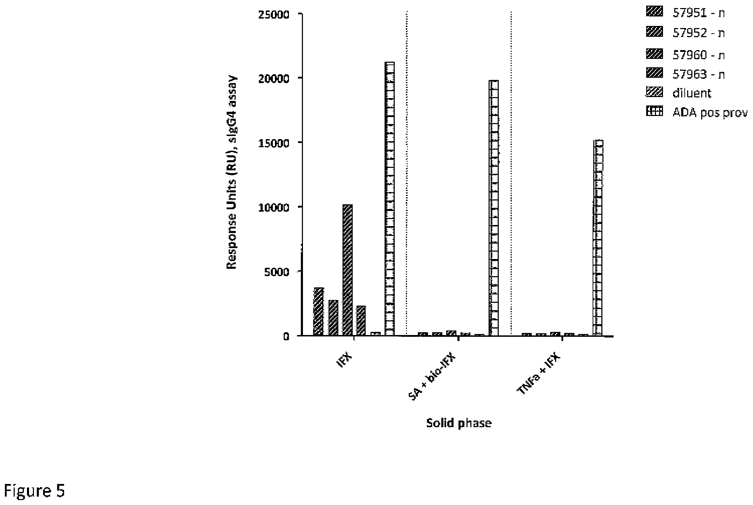

Figure 5 shows four ADA negative samples, assay diluent and one ADA positive sample tested in the ImmunoCAP™ Specific IgG4 assay as described in the experimental part below using different infliximab ImmunoCAP™test variants.
   - IFX: infliximab directly coupled to ImmunoCAP™ solid phase,
   - SA + bio-IFX: biotinylated infliximab bound to streptavidin-coupled ImmunoCAP™ according to the invention, and
   - TNFa + IFX: infliximab bound to TNF alpha-coupled ImmunoCAP™ according to the invention.

The two latter variants are examples according to the present invention attaching an IgG molecule to the solid phase via a linker for analysis of IgG4 antibodies.

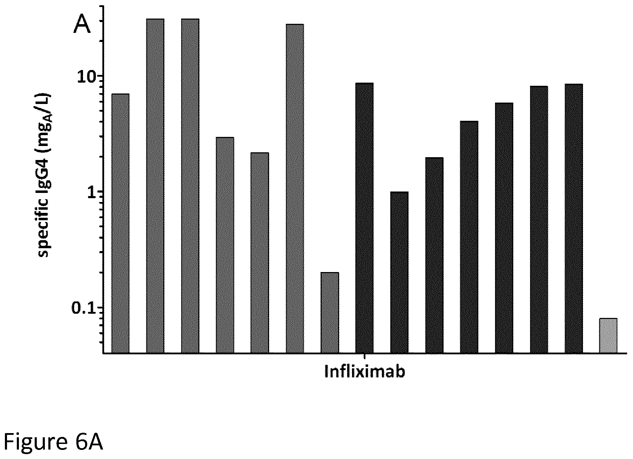

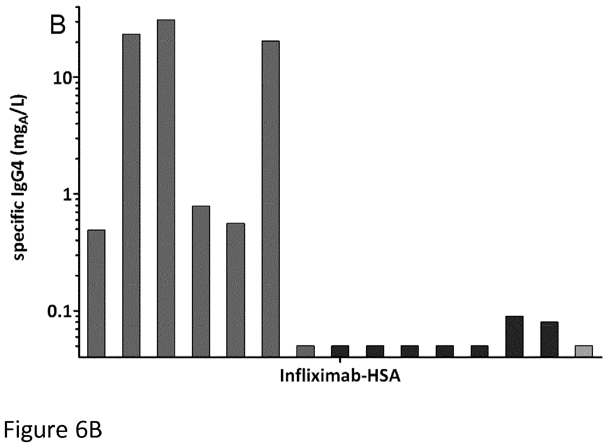

Figure 6 shows seven serum samples defined as ADA positive for infliximab using other assays (the first seven bars shown,), seven samples defined as ADA negative (the following seven bars shown), and assay diluent (the last bar, in grey) analyzed as described in Example 2 below.

### EXPERIMENTAL PART

The present examples are provided for illustrative purposes only, and should not be interpreted as any limitation of the invention in any way.

### Example 1: Assay interference caused by Fc-Fc interactions

One example of assay interference dedicated Fc-Fc interactions can be shown using therapeutic antibodies with the human IgG1 Fc domains of infliximab (a chimeric monoclonal antibody against tumour necrosis factor alpha (TNF-α) used to treat autoimmune diseases, also known as Remicade and available e.g. from JANSSEN BIOTECH INC) or adalimumab (HUMIRA ("Human Monoclonal Antibody in Rheumatoid Arthritis"), available e.g. from Abbott Labs) coupled to experimental ImmunoCAP™ tests and used in the commercial ImmunoCAP™ Specific IgG4 assay (available through www.phadia.com). The assay has an enzyme-conjugated mouse monoclonal antibody against human IgG4 as detection reagent. The detection antibody has no apparent cross-reactivity to human IgG1 (data not shown). The therapeutic antibodies are covalently coupled to the solid phase using reactive amino-groups of the therapeutic antibody and CNBr-activated groups of the cellulose sponge matrix that are placed in the ImmunoCAP™ test capsule. This results in ImmunoCAP™ tests with high background levels in the ImmunoCAP™ Specific IgG4 assay when testing samples from negative control subjects without known ADAs to infliximab or adalimumab (Table 1 below, wherein RU means Response Units; and n/a means Not Available).

**Table 1**

| | **Infliximab** | | **Adalimumab** | | **Birch pollen extract** | |
|---|---|---|---|---|---|---|
| **Sample** | **RU** | **Conc.** (mgA/L) | **RU** | **Conc.** (mgA/L) | **RU** | **Conc.** (mgA/L) |
| 57951 | 3291 | 3.2 | 4487 | 4.7 | 86 | <0.07 |
| 57952 | 2528 | 2.4 | n/a | n/a | n/a | n/a |
| 57960 | 9866 | 11.1 | 12607 | 16.4 | 417 | 0.3 |
| 57961 | 6459 | 6.7 | n/a | n/a | n/a | n/a |
| 57963 | 2019 | 1.9 | n/a | n/a | n/a | n/a |
| ADA positive | 23538 | 55.1 | n/a | n/a | n/a | n/a |

### Example 2: Coupling of infliximab to solid phase with and without linker

The length and nature of the linker has to be optimized for each solid phase and IgG molecule. The linker should be optimized for low assay background levels and preserved high signal-to-noise ratio for positive samples.

This example shows the effect of using linkers for the attachment of an IgG molecule to a solid phase of an antibody immunosorbent assay, and the results are presented in Figure 5 and 6.

The figure 5 presents results from an experiment using infliximab (IFX), a therapeutic antibody of IgG1 isotype (also known as Remicade, available e.g. from JANSSEN BIOTECH INC) attached according to the invention to a solid phase of ImmunoCAP™ (available through www.phadia.com) via a linker based on the streptavidin/biotin coupling system and via a linker based on its ligand, tumor necrosis factor alpha (TNF-α). Direct coupling of infliximab to the solid phase results in high backgrounds in the ImmunoCAP™ Specific IgG4 assay when testing negative samples while coupling of infliximab via the linkers results in low backgrounds and retained immunoreactivity of the drug. A slightly decreased signal-to-noise ratio is shown for the ImmunoCAP™ test with infliximab bound to TNF-α.

The figure 6 presents results from an experiment using infliximab (IFX) attached according to the invention to a solid phase of ImmunoCAP™ via human serum albumin (HSA) as a linker. Direct coupling of infliximab to the solid phase results in high backgrounds in the ImmunoCAP™ Specific IgG4 assay when testing negative samples (Figure 6A) while coupling of infliximab according to the invention via HSA as a linker results in low backgrounds and retained immunoreactivity of the drug (Figure 6B).

### Example 3: Variation of the size of the linker

Linkers of different sizes were provided and tested in an infliximab assay according to the invention. The results are presented in Table 2 below:

**Table 2**

| | | **Specific IgG4 assay results, infliximab (Response units)** | | | | |
|---|---|---|---|---|---|---|
| **Linker** | **MW of linker** | **Positive sample** | **Negative sample 1** | **Negative sample 2** | **Mean of Negative sample 1 and 2** | **Ratio Pos./Neg.** |
| No linker | N/A | 19615 | 6574 | 8629 | 7602 | 2.6 |
| Streptavidin | 53 kDa | 22976 | 354 | 420 | 387 | 59.4 |
| HSA | 67 kDa | 16504 | 265 | 317 | 291 | 56.7 |
| PAMAM dend. gen. 4 | 14 kDa | 23215 | 462 | 563 | 513 | 45.3 |
| PAMAM dend. gen. 0 | 0.5 kDa | 22096 | 358 | 478 | 418 | 52.9 |

### Abbreviations:

MW = Molecular weight
N/A = Not applicable
HSA = Human serum albumin
PAMAM dend. gen. 4 = PAMAM dendrimer generation 4
PAMAM dend. gen. 0 = PAMAM dendrimer generation 0

## Claims

1. A method for reducing unspecific binding caused by Fc-Fc interactions in an immunoassay, wherein one or more anti-drug antibodies (ADAs) of IgG4 isotype are analyzed in a solution by
a) contacting the solution with a solid phase to which IgG molecules capable of being bound by said ADAs have been attached;
b) allowing said ADAs to specifically bind to the IgG molecule(s), and optionally removing any excess of solution;
c) adding a labeled IgG4-specific antibody capable of specific binding of the ADAs;
d) removing any excess of reagent; and
e) detecting label bound or unbound to determine directly or indirectly the presence or concentration of the ADAs in the solution,
wherein in step a), the IgG molecule(s) have been separated from the solid phase via a linker, and wherein said linker reduces the extent of solid phase-induced changes in the structure of the attached IgG molecules that facilitate Fc-Fc interactions between the attached IgG molecules and IgG4-specific antibody by distancing the attached IgG molecules from the proximity of the solid phase.

2. A method according to claim 1, wherein the ADAs are directed against an IgG1 drug.

3. A method according to claim 1, wherein the IgG molecules are selected from the group consisting of IgG1, IgG2, IgG3 and IgG4.

4. A method according to any one of the preceding claims, wherein the linker is an organic molecule, an amino acid, a peptide, a protein or molecule of protein origin, a monosaccharide, an oligosaccharide or a polysaccharide.

5. A method according to any one of the preceding claims, wherein the linker is formed by covalent coupling of the IgG molecule(s) to molecules extending from a solid phase comprised of natural polymer, such as cellulose.

6. A method according to any one of claims 1-5, wherein the IgG molecule(s) is an antibody with known specificity and the linker is its target ligand.

7. A method according to any one of claims 1-5, wherein the IgG molecule(s) have been labeled with biotin and the linker is streptavidin.

8. A method according to any one of claims 1-5, wherein the the IgG molecule(s) is at least one fusion protein between a ligand-binding molecule and the Fc region of the IgG molecule, and the linker is its target ligand.

9. A method according to any one of the preceding claims, wherein the labeled IgG4-specific antibody capable of binding the endogenously formed antibodies is a monoclonal or polyclonal antibody.

10. A method according to any one of the preceding claims, wherein the solution is a biological sample.

11. Use of a method according to any one of claims 1-10 to monitor a patient's antibody response to a therapeutic antibody.

12. Use of a method according to any one of claims 1-10 in drug development.

## Patentansprüche

1. Verfahren zur Verringerung unspezifischer Bindung, die durch Fc-Fc-Wechselwirkungen hervorgerufen wird, in einem Immunassay, wobei ein oder mehrere Anti-Arzneistoff-Antikörper (Anti-Drug Antibodies - ADAs) des IgG4-Isotyps in einer Lösung durch Folgendes analysiert werden
a) Inkontaktbringen der Lösung mit einer Festphase, an der IgG-Moleküle angebracht worden sind, die von den ADAs gebunden werden können;
b) Ermöglichen, dass die ADAs spezifisch an das/die IgG-Molekül(e) binden und gegebenenfalls Entfernen überschüssiger Lösung;
c) Zugeben eines markierten IgG4-spezifischen Antikörpers, der zur spezifischen Bindung der ADAs in der Lage ist;
d) Entfernen von überschüssigem Reagens; und
e) Nachweisen von gebundenem oder ungebundenem Marker, um direkt oder indirekt das Vorhandensein oder die Konzentration der ADAs in der Lösung zu bestimmen,
wobei in Schritt a) das/die IgG-Molekül(e) über einen Linker von der Festphase separiert ist/sind und wobei der Linker das Ausmaß von festphaseninduzierten Veränderungen in der Struktur der angebrachten IgG-Moleküle verringert, die Fc-Fc-Wechselwirkungen zwischen den angebrachten IgG-Molekülen und dem IgG4-spezifischen Antikörper erleichtern, indem die angebrachten IgG-Moleküle von der Nähe der Festphase beabstandet werden.

2. Verfahren nach Anspruch 1, wobei die ADAs gegen einen IgG1-Arzneistoff gerichtet sind.

3. Verfahren nach Anspruch 1, wobei die IgG-Moleküle aus der Gruppe bestehend aus IgG1, IgG2, IgG3 und IgG4 ausgewählt sind.

4. Verfahren nach einem der vorhergehenden Ansprüche, wobei der Linker ein organisches Molekül, eine Aminosäure, ein Peptid, ein Protein oder ein Molekül mit Proteinursprung, ein Monosaccharid, ein Oligosaccharid oder ein Polysaccharid ist.

5. Verfahren nach einem der vorhergehenden Ansprüche, wobei der Linker durch kovalente Kopplung des IgG-Moleküls/der IgG-Moleküle an Moleküle gebildet wird, die sich von einer Festphase erstrecken, die aus einem natürlichen Polymer, wie Cellulose, besteht.

6. Verfahren nach einem der Ansprüche 1 bis 5, wobei das/die IgG-Molekül(e) ein Antikörper mit bekannter Spezifität ist/sind und der Linker dessen Zielligand ist.

7. Verfahren nach einem der Ansprüche 1-5, wobei das/die IgG-Molekül(e) mit Biotin markiert wurden und der Linker Streptavidin ist.

8. Verfahren nach einem der Ansprüche 1-5, wobei das/die IgG-Molekül(e) mindestens ein Fusionsprotein zwischen einem ligandenbindenden Molekül und der Fc-Region des IgG-Moleküls ist/sind und der Linker dessen Zielligand ist.

9. Verfahren nach einem der vorhergehenden Ansprüche, wobei der markierte IgG4-spezifische Antikörper, der in der Lage ist, die endogen gebildeten Antikörper zu binden, ein monoklonaler oder polyklonaler Antikörper ist.

10. Verfahren nach einem der vorhergehenden Ansprüche, wobei die Lösung eine biologische Probe ist.

11. Verwendung eines Verfahrens nach einem der Ansprüche 1-10 zur Überwachung der Antikörperantwort eines Patienten auf einen therapeutischen Antikörper.

12. Verwendung eines Verfahrens nach einem der Ansprüche 1-10 in der Arzneistoffentwicklung.

## Revendications

1. Procédé pour réduire une liaison non spécifique provoquée par des interactions Fc-Fc dans un immunodosage, dans lequel un ou plusieurs anticorps anti-médicament (ADA) d'isotype IgG4 sont analysés dans une solution en
a) mettant en contact la solution avec une phase solide à laquelle des molécules d'IgG susceptibles d'être liées par lesdits ADA ont été fixées ;
b) permettant aux ADA de se lier spécifiquement à la ou aux molécules d'IgG, et éventuellement d'éliminer tout excès de solution ;
c) ajoutant un anticorps spécifique marqué par IgG4 capable de se lier spécifiquement aux ADA ;
d) éliminant tout excès de réactif ; et
e) détectant le marqueur lié ou non lié pour déterminer directement ou indirectement la présence ou la concentration des ADA dans la solution,
dans lequel dans l'étape a), la ou les molécules d'IgG ont été séparées de la phase solide par un linker, et dans lequel ledit linker réduit l'étendue des changements induits en phase solide dans la structure des molécules d'IgG attachées qui facilitent les interactions Fc-Fc entre les molécules d'IgG attachées et l'anticorps IgG4 spécifique en distanciant les molécules d'IgG attachées de la proximité de la phase solide.

2. Procédé selon la revendication 1, dans lequel les ADA sont dirigés contre un médicament IgG1.

3. Procédé selon la revendication 1, dans lequel les molécules d'IgG sont choisies dans le groupe constitué par IgG1, IgG2, IgG3 et IgG4.

4. Procédé selon l'une quelconque des revendications précédentes, dans lequel le lieur est une molécule organique, un acide aminé, un peptide, une protéine ou une molécule d'origine protéique, un monosaccharide, un oligosaccharide ou un polysaccharide.

5. Procédé selon l'une quelconque des revendications précédentes, dans lequel le lieur est formé par couplage covalent de la ou des molécules d'IgG à des molécules s'étendant à partir d'une phase solide constituée de polymère naturel, tel que la cellulose.

6. Procédé selon l'une quelconque des revendications 1 à 5, dans lequel la ou les molécules d'IgG sont un anticorps avec une spécificité connue et le lieur est son ligand cible.

7. Procédé selon l'une quelconque des revendications 1 à 5, dans lequel la ou les molécules d'IgG ont été marquées avec de la biotine et le lieur est de la streptavidine.

8. Procédé selon l'une quelconque des revendications 1 à 5, dans lequel la ou les molécules d'IgG sont au moins une protéine de fusion entre une molécule liant le ligand et la région Fc de la molécule IgG, et le lieur est son ligand cible.

9. Procédé selon l'une quelconque des revendications précédentes, dans lequel l'anticorps spécifique d'IgG4 marqué capable de se lier aux anticorps formés de manière endogène est un anticorps monoclonal ou polyclonal.

10. Procédé selon l'une quelconque des revendications précédentes, dans lequel la solution est un échantillon biologique.

11. Utilisation d'un procédé selon l'une quelconque des revendications 1 à 10 pour surveiller la réponse d'anticorps d'un patient à un anticorps thérapeutique.

12. Utilisation d'un procédé selon l'une quelconque des revendications 1 à 10 dans le développement de médicaments.
